(19) Europäisches Patentamt
European Patent Office
Office européen des brevets

(11) EP 0 937 711 A1

# (12) EUROPÄISCHE PATENTANMELDUNG

(43) Veröffentlichungstag:
**25.08.1999 Patentblatt 1999/34**

(51) Int. Cl.[6]: **C07C 327/48**, A61K 31/165, A61K 31/18

(21) Anmeldenummer: **98102751.9**

(22) Anmeldetag: **18.02.1998**

(84) Benannte Vertragsstaaten:
**AT BE CH DE DK ES FI FR GB GR IE IT LI LU MC NL PT SE**
Benannte Erstreckungsstaaten:
**AL LT LV MK RO SI**

(71) Anmelder: **Roche Diagnostics GmbH**
**68298 Mannheim (DE)**

(72) Erfinder:
- **Kucznierz, Ralf, Dr.**
  **67098 Bad Dürkheim (DE)**
- **Grams, Frank, Dr.**
  **68219 Mannheim (DE)**
- **Leinert, Herbert, Dr.**
  **64646 Heppenheim (DE)**
- **von der Saal, Wolfgang, Dr.**
  **69469 Weinheim (DE)**
- **Stegmeier, Karlheinz, Dr.**
  **64646 Heppenheim (DE)**

## (54) Neue Thiobenzamide, Verfahren zu ihrer Herstellung sowie diese enthaltende Arzneimittel

(57) Die Erfindung betrifft neue Thiobenzamide der allgemeinen Formel I

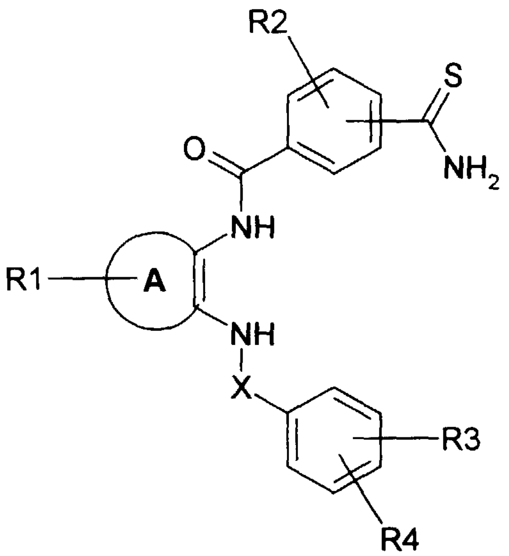

(I)

in der $R^1$ bis $R^4$, die in der Beschreibung angegebene Bedeutung haben, sowie deren Hydrate, Solvate und physiologisch verträgliche Salze, optisch aktive Formen, Racemate und Diastereomerengemische, Verfahren zu ihrer Herstellung sowie Arzneimittel, die diese Verbindungen enthalten zur Behandlung thromboembolischer Erkrankungen.

EP 0 937 711 A1

## Beschreibung

Die Erfindung betrifft neue Thiobenzamide der allgemeinen Formel I

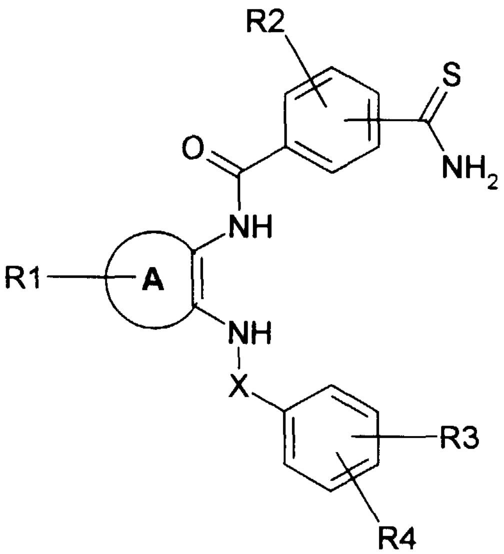

(I)

in der

$R^1$ ein Wasserstoffatom, ein Halogenatom, eine Hydroxygruppe, eine Aminogruppe, eine Nitrogruppe, eine Carboxylgruppe, eine Carbamoylgruppe, eine Thiocarbamoylgruppe, eine Alkylgruppe, eine Cycloalkylgruppe, eine Alkenylgruppe, eine Alkinylgruppe, eine Alkoxygruppe, eine Hydroxyalkylgruppe, eine Alkoxyalkylgruppe, eine Aralkyloxygruppe, eine Alkenyloxygruppe, eine Alkinyloxygruppe, eine Carboxyalkylgruppe, eine Alkoxycarbonylgruppe, eine Alkenyloxycarbonylgruppe, eine Alkinyloxycarbonylgruppe, eine Alkyloxycarbonylalkylgruppe, eine Alkenyloxycarbonylalkylgruppe oder eine Alkinyloxycarbonylalkylgruppe sein können;

$R^2$ ein Wasserstoffatom, ein Halogenatom, eine Hydroxygruppe, eine Aminogruppe, eine Nitrogruppe, eine Carboxylgruppe, eine Carbamoylgruppe, eine Alkylgruppe, eine Cycloalkylgruppe, eine Alkenylgruppe, eine Alkinylgruppe, eine Alkoxygruppe, eine Hydroxyalkylgruppe, eine Alkoxyalkylgruppe, eine Aralkyloxygruppe, eine Alkenyloxygruppe, eine Alkinyloxygruppe, eine Carboxyalkylgruppe, eine Alkoxycarbonylgruppe, eine Alkenyloxycarbonylgruppe, eine Alkinyloxycarbonylgruppe, eine Alkyloxycarbonylalkylgruppe, eine Alkenyloxycarbonylalkylgruppe oder eine Alkinyloxycarbonylalkylgruppe sein können;

$R^3$, $R^4$ gleich oder verschieden sind und ein Wasserstoffatom, ein Halogenatom, eine Hydroxygruppe, eine Aminogruppe, eine Nitrogruppe, eine Carboxylgruppe, eine Carbamoylgruppe, eine Thiocarbarmoylgruppe, eine Alkylgruppe, eine Cycloalkylgruppe, eine Alkenylgruppe, eine Alkinylgruppe, eine Alkoxygruppe, eine Hydroxyalkylgruppe, eine Alkoxyalkylgruppe, eine Aralkyloxygruppe, eine Alkenyloxygruppe, eine Alkinyloxygruppe, eine Carboxyalkylgruppe, eine Alkoxycarbonylgruppe, eine Alkenyloxycarbonylgruppe, eine Alkinyloxycarbonylgruppe, eine Alkyloxycarbonylalkylgruppe, eine Alkenyloxycarbonylalkylgruppe oder eine Alkinyloxycarbonylalkylgruppe sein können oder $R^3$ und $R^4$ zusammen mit dem Arylrest, an den sie gebunden sind einen kondensierten Aromaten wie einen Naphthylrest, einen Chinolinrest oder einen Isochinolinrest bilden;

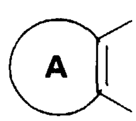

eines der aromatischen Fragmente Phenylen, Thiophen-2,3-diyl, Thiophen-3,4-diyl, Furan-2,3-diyl, Furan-3,4-diyl, Pyridin-2,3-diyl, Pyridin-3,4-diyl, Pyridin-5,6-diyl, Pyridazin-3,4-diyl oder Pyridazin-4,5-diyl bedeutet und

X eine geradkettige oder verzweigte Alkylengruppe, eine Carbonylgruppe oder eine $SO_2$-Gruppe bedeutet,

sowie Hydrate, Solvate und physiologisch verträgliche Salze davon. Gegenstand der Erfindung sind auch die optisch aktiven Formen, die Racemate und die Diastereomerengemische dieser Verbindungen.

Die Erfindung betrifft auch Verfahren zur Herstellung der obigen Verbindungen, Arzneimittel, die solche Verbindungen enthalten, sowie die Verwendung dieser Verbindungen bei der Herstellung von Arzneimitteln.

Die Thiobenzamide der allgemeinen Formel I, ihre Solvate und ihre Salze greifen durch reversible Inhibierung von Faktor Xa in den Prozeß der Blutgerinnung ein und verhindern somit die Entstehung von Gerinnungsthromben. Sie können deshalb bei der Bekämpfung und Verhütung von Krankheiten, wie Thrombose, Apoplexie, Herzinfarkt, Entzündungen und Arteriosklerose, verwendet werden.

Faktor Xa ist eine Serinprotease des Gerinnungssystems, die die proteolytische Umwandlung von Prothrombin in Thrombin katalysiert. Thrombin, als letztes Enzym der Gerinnungskaskade, spaltet einerseits Fibrinogen zu Fibrin, das nach Quervernetzung mittels Faktor XIIIa zu einem unlöslichen Gel wird und die Matrix für einen Thrombus bildet, andererseits aktiviert es durch Proteolyse seines Rezeptors auf den Blutplättchen die Plättchenaggregation und trägt auf diesem Weg ebenfalls zur Thrombusbildung bei. Bei der Verletzung eines Blutgefäßes sind diese Prozesse notwendig, um eine Blutung zu stoppen. Unter normalen Umständen sind keine meßbaren Thrombin-Konzentrationen im Blutplasma vorhanden. Ein Ansteigen der Thrombinkonzentration kann zur Ausbildung von Thromben und damit zu thromboembolischen Krankheiten führen, die vor allem in den Industriestaaten sehr häufig auftreten. Durch Hemmung von Faktor Xa kann die Entstehung von Thrombin verhindert werden.

Kürzlich wurde berichtet, daß Amidinoarylpropansäure-Derivate wie (+)-(2S)-2-[4-[[(3S)-1-Acetimidoyl-3-pyrrolidinyl]oxy]phenyl]-3-(7-amidino-2-naphthyl]propansäurehydrochlorid-pentahydrat (DX-9065a; Formel IIa) Faktor Xa inhibieren (*J. Med. Chem.* **1994**, *37*, 1200-1207; *Thrombosis and Haemostasis* **1994**, *71*, 314-319; EP-0-540-051-A-1). Weitere bekannte Faktor-Xa-Inhibitoren sind 1,2-Bis-(5-amidino-2-benzofuranyl)-ethan (DABE, Formel IIb, *Thrombosis Research* **1980**, *19*, 339-349) oder auch Phenylamino-methyl-naphthamidine der allgemeinen Formel IIc (WO96/16940).

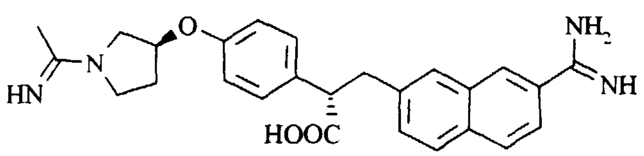

(IIa)

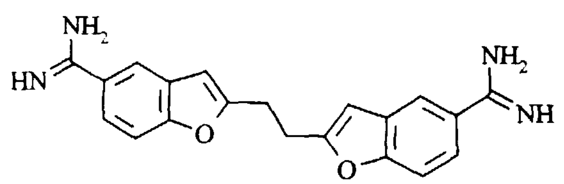

(IIb)

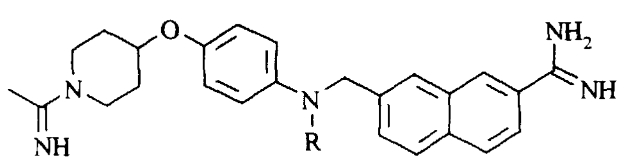

(IIc)

Die erfindungsgemäßen, neuen Thiobenzamide der allgemeinen Formel I sowie Hydrate, Solvate und physiologisch verträgliche Salze davon sind potente und selektive Faktor Xa-Inhibitoren.

Bedeutet $R^1$, $R^2$ in der allgemeinen Formel I ein Halogenatom, so kann dies jeweils ein Fluor-, Chlor-, Brom- oder Iodatom bedeuten, bevorzugt sind jedoch Fluor-, Chlor- oder Bromsubstituenten.

Bedeutet $R^1$, $R^2$ in der allgemeinen Formel I eine Alkylgruppe, so kann diese geradkettig oder verzweigt sein und 1 bis 8 Kohlenstoffatome enthalten. Bevorzugt sind die Methyl-, Ethyl-, *n*-Propyl-, *i*-Propyl-, *n*-Butyl-, *i*-Butyl-, *t*-Butyl-, Pentyl- und die Hexylgruppe

Bedeutet $R^1$, $R^2$, $R^3$, $R^4$ in der allgemeinen Formel I eine Cycloalkylgruppe, so kann diese substituiert oder unsubstituiert sein und 3 bis 8 Kohlenstoffatome enthalten. Bevorzugt sind die Cyclopropyl-, Cyclopentyl-, Cyclohexyl- und die Cyclooctylgruppe.

Bedeutet $R^1$, $R^2$, $R^3$, $R^4$ in der allgemeinen Formel I eine Alkenylgruppe, so kann diese geradkettig oder verzweigt sein und 2 bis 8 Kohlenstoffatome enthalten. Bevorzugt sind die Vinyl-, 1-Propenyl-, 2-Propenyl-, 2-Methyl-2-propenyl-, 1-Butenyl-, 1-Pentenyl- und die 1-Hexenylgruppe.

Bedeutet $R^1$, $R^2$, $R^3$, $R^4$ in der allgemeinen Formel I eine Alkinylgruppe, so kann diese geradkettig oder verzweigt sein und 2 bis 8 Kohlenstoffatome enthalten. Bevorzugt sind die Ethinyl- und die Propargylgruppe.

Eine Alkoxygruppe als Substituent $R^1$, $R^2$, $R^3$, $R^4$ in der allgemeinen Formel I enthält 1 bis 8 Kohlenstoffatome und ist geradkettig oder verzweigt. Bevorzugt sind die Methoxy-, Ethoxy-, *n*-Propyloxy-, *i*-Propyloxy-, *n*-Butyloxy-, *i*-Butyloxy-, *tert.*-Butyloxy-, Pentyloxy- und die Hexyloxygruppe.

Bedeutet $R^1$, $R^2$, $R^3$, $R^4$ in der allgemeinen Formel I eine Hydroxyalkylgruppe, so kann diese geradkettig oder verzweigt sein und 1 bis 8 Kohlenstoffatome enthalten. Bevorzugt sind die Hydroxymethyl-, Hydroxyethyl-, Hydroxypropyl-, Hydroxybutyl-, Hydroxypentyl- und die Hydroxyhexylgruppe.

Bedeutet $R^1$, $R^2$, $R^3$, $R^4$ in der allgemeinen Formel I eine Alkoxyalkylgruppe, so sind unter den betreffenden Alkylresten jeweils geradkettige oder verzweigte Alkylketten mit 1 bis 8 Kohlenstoffatomen, zu verstehen. Bevorzugt sind die Methoxymethyl-, Ethoxymethyl-, Methoxyethyl- und die Ethoxyethylgruppe.

Bedeutet $R^1$, $R^2$, $R^3$, $R^4$ in der allgemeinen Formel I eine Aralkyloxygruppe, so enthält diese eine mit einer geradkettigen oder verzweigten $C_1$-$C_8$-Alkylkette verknüpfte Phenylgruppe, eine mit einer geradkettigen oder verzweigten $C_1$-$C_8$-Alkylkette verknüpfte Naphthylgruppe oder eine mit einer geradkettigen oder verzweigten $C_1$-$C_8$-Alkylkette verknüpfte Biphenylgruppe. Bevorzugt sind dabei die Benzyloxygruppe, die p-Phenylbenzyloxygruppe und die Naphthylmethyloxygruppe.

Eine Alkenyloxygruppe als Substituent $R^1$, $R^2$, $R^3$, $R^4$ in der allgemeinen Formel I enthält 3 bis 8 Kohlenstoffatome und ist geradkettig oder verzweigt. Bevorzugt sind die Vinyloxy- und Allyloxygruppe.

Eine Alkinyloxygruppe als Substituent $R^1$, $R^2$, $R^3$, $R^4$ in der allgemeinen Formel I enthält 3 bis 8 Kohlenstoffatome und ist geradkettig oder verzweigt. Bevorzugt ist die Propargyloxygruppe.

Eine Carboxyalkylgruppe als Substituent $R^1$, $R^2$, $R^3$, $R^4$ in der allgemeinen Formel I enthält Alkyle mit 1 bis 8 Kohlenstoffatomen, die jeweils geradkettig oder verzweigt sein können. Bevorzugt sind die Carboxymethyl-, die Carboxyethyl- sowie die Carboxypropylgruppe.

Eine Alkoxycarbonylgruppe als Substituent $R^1$, $R^2$, $R^3$, $R^4$ in der allgemeinen Formel I enthält eine geradkettige oder verzweigte Alkylkette mit 1 bis 8 Kohlenstoffatomen. Bevorzugt sind die Methoxycarbonyl- und die Ethoxycarbonylgruppe sowie die *i*-Propyloxycarbonyl- und die *tert.*-Butyloxycarbonylgruppe.

Bedeutet $R^1$, $R^2$, $R^3$, $R^4$ in der allgemeinen Formel I eine Alkenyloxycarbonylgruppe, so enthält diese eine geradkettige oder verzweigte Alkenylkette mit 3 bis 8 Kohlenstoffatomen. Bevorzugt ist die Allyloxycarbonylgruppe.

Bedeutet $R^1$, $R^2$, $R^3$, $R^4$ in der allgemeinen Formel I eine Alkinyloxycarbonylgruppe, so enthält diese eine geradkettige oder verzweigte Alkinylkette mit 3 bis 8 Kohlenstoffatomen. Bevorzugt ist die Propargyloxycarbonylgruppe.

Bedeutet $R^1$, $R^2$, $R^3$, $R^4$ in der allgemeinen Formel I eine Alkyloxycarbonylalkylgruppe, so sind unter den Alkylresten jeweils geradkettige oder verzweigte Alkylketten mit 1 bis 8 Kohlenstoffatomen, zu verstehen. Bevorzugt sind die Methoxycarbonylmethyl-, Ethoxycarbonylmethyl-, Methoxycarbonylethyl-, Ethoxycarbonylethyl-, Methoxycarbonylpropyl- und die Ethoxycarbonylpropylgruppe.

Bedeutet $R^1$, $R^2$, $R^3$, $R^4$ in der allgemeinen Formel I eine Alkenyloxycarbonylalkylgruppe, so ist der Alkenylrest geradkettig oder verzweigt mit 3 bis 8 Kohlenstoffatomen und die Alkylkette geradkettig oder verzweigt mit 1 bis 8 Kohlenstoffatomen. Bevorzugt sind die Allyloxycarbonylmethyl-, Allyloxycarbonylethyl- und die Allyloxycarbonylpropylgruppe.

Bedeutet $R^1$, $R^2$, $R^3$, $R^4$ in der allgemeinen Formel I eine Alkinyloxycarbonylalkylgruppe, so ist der Alkinylrest geradkettig oder verzweigt mit 3 bis 8 Kohlenstoffatomen und die Alkylkette geradkettig oder verzweigt mit 1 bis 8 Kohlenstoffatomen. Bevorzugt sind die Propargyloxycarbonylmethyl-, Propargyloxycarbonylethyl- und die Propargyloxycarbonylpropylgruppe.

Bedeutet $R^1$, $R^2$, $R^3$, $R^4$ in der allgemeinen Formel I eine Aminogruppe, so kann diese unsubstituiert oder auch substituiert sein und zwar mit einer oder zwei $C_1$-$C_6$-Alkylgruppen, vorzugsweise Methyl oder Ethyl, mit einer oder zwei $C_3$-$C_8$-Cycloalkylgruppen, vorzugsweise Cyclopropyl, Cyclopentyl, Cyclohexyl oder Cyclooctyl, mit einer oder zwei $C_1$-$C_6$-Hydroxyalkylgruppen, vorzugsweise Hydroxyethyl oder Hydroxypropyl, mit einer oder zwei $C_3$-$C_6$-Alkenylgruppen, vorzugsweise Allyl, mit einer oder zwei $C_3$-$C_6$-Alkinylgruppen, vorzugsweise Propargyl oder mit einer oder zwei Aralkylgruppen, vorzugsweise Benzyl. Die Spezifizierung ($C_1$-$C_6$) steht hier jeweils für eine geradkettige oder verzweigte Alkylkette mit 1 bis 6 Kohlenstoffatomen, ($C_3$-$C_8$) bezeichnet eine verzweigte oder unverzweigte Cycloalkylgruppe mit 3 bis 8 Kohlenstoffatomen und ($C_3$-$C_6$) kennzeichnet wahlweise eine verzweigte oder unverzweigte Alkenyl- oder Alkinylgruppierung mit 3 bis 6 Kohlenstoffatomen.

In der allgemeinen Formel I können die Substituenten $R^3$ und $R^4$ gleich oder verschiedenartig sein.

Halogene als Substituenten $R^3$, $R^4$ können Fluor-, Chlor-, Brom- und Iodatome, bevorzugt jedoch Chlor- oder Bromatome sein.

Bedeutet $R^3$, $R^4$ in der allgemeinen Formel I eine Alkylgruppe, so kann diese geradkettig oder verzweigt sein und 1 bis 6 Kohlenstoffatome enthalten. Bevorzugt sind die Methyl-, Ethyl-, *n*-Propyl-, *i*-Propyl-, *n*-Butyl-, *i*-Butyl-, *t*-Butyl-, Pentyl- und die Hexylgruppe.

Bedeuten $R^3$ und $R^4$ in der allgemeinen Formel I zusammen mit dem Arylrest, an den sie gebunden sind, einen kondensierten Aromaten, so versteht darunter einen Naphthylrest, einen Chinolinrest oder einen Isochinolinrest. Der kondensierte Aromat kann unsubstituiert sein oder gegebenenfalls einen oder mehrere $C_1$-$C_8$-Alkylsubstituenten, vorzugsweise Methyl, einen oder mehrere $C_1$-$C_8$-Alkyloxysubstituenten, vorzugsweise Methoxy, eine oder mehrere Carboxylgruppen, einen oder mehrere $C_1$-$C_8$-Alkoxycarbonylsubstituenten, vorzugsweise Methoxycarbonyl oder Ethoxycarbonyl, oder einen oder mehrere Halogensubstituenten tragen. Die Spezifizierung $C_1$-$C_8$ steht hier jeweils für eine geradkettige oder verzweigte Alkylkette mit 1 bis 8 Kohlenstoffatomen Halogene als Substituenten des kondensierten Aromaten können Fluor-, Chlor-, Brom- und Iodatome, bevorzugt jedoch Fluor-, Chlor- oder Bromatome sein.

Das Fragment

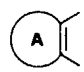

bedeutet eines der aromatischen Fragmente Phenylen, Thiophen-2,3-diyl, Thiophen-3,4-diyl, Furan-2,3-diyl, Furan-3,4-diyl, Pyridin-2,3-diyl, Pyridin-3,4-diyl, Pyridin-5,6-diyl, Pyridazin-3,4-diyl oder Pyridazin-4,5-diyl. Bevorzugt sind Phenylen, Thiophen-2,3-diyl, Thiophen-3,4-diyl, Pyridin-2,3-diyl, Pyridin-3,4-diyl und Pyridin-5,6-diyl.

Die Gruppe X bedeutet wahlweise eine Alkylengruppe, eine Carbonylgruppe oder das Fragment $SO_2$. Bedeutet X in der allgemeinen Formel I eine Alkylengruppe, so kann diese geradkettig oder verzweigt sein und 1 bis 8 Kohlenstoffatome enthalten. Bevorzugt ist die Methylengruppe.

Besonders bevorzugt sind Verbindungen der allgemeinen Formel I, in denen

$R^1$ ein Wasserstoffatom, ein Chloratom, eine Hydroxygruppe, eine Carboxylgruppe, eine Methylgruppe, eine Ethylgruppe, eine *tert.*-Butylgruppe, eine Methoxygruppe, eine Ethoxygruppe, eine *tert.*-Butyloxygruppe, eine Benzyloxygruppe, eine Allyloxygruppe, eine Methoxycarbonylgruppe, eine Ethoxycarbonylgruppe oder eine Allyloxycarbonylgruppe bedeuten;

$R^2$ ein Wasserstoffatom, ein Chloratom, eine Hydroxygruppe, eine Carboxylgruppe, eine Thiocarbamoylgruppe, eine Methylgruppe, eine Ethylgruppe, eine *tert.*-Butylgruppe, eine Methoxygruppe, eine Ethoxygruppe, eine *tert.*-Butyloxygruppe, eine Benzyloxygruppe, eine Allyloxygruppe, eine Methoxycarbonylgruppe, eine Ethoxycarbonylgruppe oder eine Allyloxycarbonylgruppe bedeuten;

$R^3$, $R^4$ gleich oder verschieden sind und ein Wasserstoffatom, ein Chloratom, eine Hydroxygruppe, eine Carboxylgruppe, eine Thiocarbamoylgruppe, eine Methylgruppe, eine Ethylgruppe, eine *tert.*-Butylgruppe, eine Methoxygruppe, eine Ethoxygruppe, eine *tert.*-Butyloxygruppe, eine Benzyloxygruppe, eine Allyloxygruppe, eine Methoxycarbonylgruppe, eine Ethoxycarbonylgruppe oder eine Allyloxycarbonylgruppe bedeuten oder $R^3$ und $R^4$ zusammen mit dem Arylrest, an den sie gebunden sind einen Naphthylrest bilden;

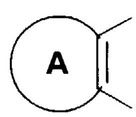

eines der aromatischen Fragmente Phenylen, Pyridin-2,3-diyl, Pyridin-3,4-diyl, Pyridin-5,6-diyl bedeutet und

X eine Carbonylgruppe oder eine $SO_2$-Gruppe bedeutet.

Insbesonders bevorzugt sind Verbindungen der allgemeinen Formel I in denen $R^1$ Wasserstoff Carboxyl oder Methoxycarbonyl bedeuten, $R^2$ Wasserstoff $R^3$, $R^4$ gleich oder verschieden sind und Wasserstoff Carboxyl, Thiocarbamoyl oder Methoxy bedeuten oder $R^3$ und $R^4$ zusammen mit dem Arylrest, an den sie gebunden sind einen Naphthylrest bilden, X eine Carbonylgruppe oder eine $SO_2$-Gruppe bedeutet und

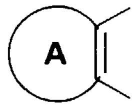

Phenylen bedeutet.

Unter den physiologisch verträglichen Salzen der allgemeinen Formel I versteht man beispielsweise Formiate, Acetate, Caproate, Oleate, Lactate oder Salze von Carbonsäuren mit bis zu 18 Kohlenstoffatomen oder Salze von Dicarbonsäuren und Tricarbonsäuren wie Citrate, Malonate und Tartrate oder Alkansulfonate mit bis zu 10 Kohlenstoffatomen oder p-Toluolsulfonate oder Salicylate oder Trifluoracetate oder Salze von physiologisch verträglichen Mineralsäuren wie Salzsäure, Bromwasserstoffsäure, Iodwasserstoffsäure, Schwefelsäure, Phosphorsäure. Die Verbindungen der Formel I mit einer oder zwei freien Säuregruppen am Phosphonatfragment können auch Salze mit physiologisch verträglichen Basen bilden. Beispiele solcher Salze sind Alkalimetall-, Erdalkalimetall-, Ammonium- und Alkylammoniumsalze, wie das Natrium-, Kalium-, Calcium- oder Tetramethylammoniumsalz.

Die Verbindungen der Formel I können solvatisiert, insbesondere hydratisiert sein. Die Hydratisierung kann im Zuge der Herstellung erfolgen oder allmählich als Folge hygroskopischer Eigenschaften einer zunächst wasserfreien Verbindung der Formel I auftreten.

Gegenstand der Erfindung sind auch die optisch aktiven Formen, die Racemate und die Diastereomerengemische von Verbindungen der allgemeinen Formel I.

Zur Herstellung von Arzneimitteln werden die Substanzen der allgemeinen Formel I mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, z.B. in Olivenöl, suspendiert oder gelöst.

Die Substanzen der allgemeinen Formel I und ihre Salze können in flüssiger oder fester Form oral, enteral oder parenteral appliziert werden. Bevorzugt ist die orale Applikationsform. Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Komplexbildner (wie Ethylendiamintetraessigsäure und deren untoxische Salze) und hochmolekulare Polymere wie flüssiges Polyethylenoxid zur Viskositätsregulierung. Feste Trägerstoffe sind z.B. Stärke, Lactose, Mannit, Methylcellulose, Talcum, hochdisperse Kieselsäuren, hochmolekulare Fettsäuren (wie Stearinsäure), tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die Verbindungen werden üblicherweise in Mengen von 1-1500 mg pro Tag bezogen auf 75 kg Körpergewicht appliziert. Bevorzugt ist es, 2-3 mal pro Tag 1-2 Tabletten mit einem Wirkstoffgehalt von 1-500 mg zu verabreichen. Die Tabletten können auch retardiert sein, wodurch nur noch einmal pro Tag 1-2 Tabletten mit 2-700 mg Wirkstoff gegeben werden müssen. Der Wirkstoff kann auch durch Injektion 1-8 mal pro Tag oder durch Dauerinfusion gegeben werden, wobei 5-2000 mg pro Tag normalerweise ausreichen.

Die Herstellung von Verbindungen der allgemeinen Formel I erfolgt nach an sich bekannten Methoden.

Die Verbindungen der allgemeinen Formel I werden hergestellt, indem man beispielsweise eine Verbindung der allgemeinen Formel III

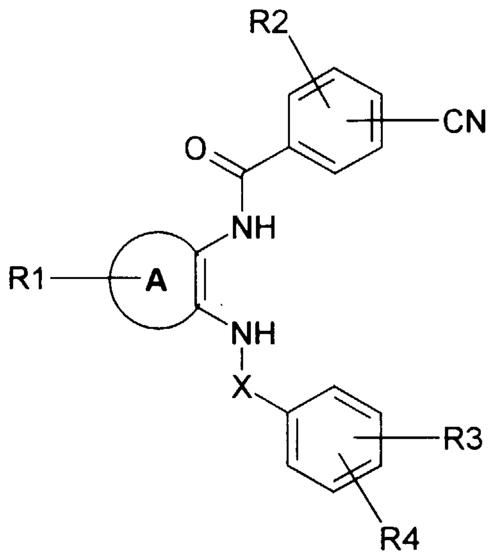

(III)

in der $R^1$, $R^2$, $R^3$, $R^4$, X und

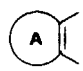

die oben angegebenen Bedeutungen besitzen, mit Schwefelwasserstoff in einem inerten Lösungsmittel wie z. B. Pyridin, Ethanol, Methanol oder N,N-Dimethylformamid bei Temperaturen zwischen 0°C und Siedepunkt des Lösungsmittels, vorzugsweise bei 0 bis 30°C in Gegenwart einer Hilfsbase wie z. B. Triethylamin, N-Methylmorpholin, Ethyldiisopropylamin oder in Gegenwart von gesättigter ethanolischer Ammoniaklösung zur Reaktion bringt. Anstelle von Schwefelwasserstoff können auch andere Sulfidierungsreagenzien wie Ammoniumsulfid, Natriumsulfid / Trimethylchlorsilan, Natriumtrimethylsilylsulfid, Bis-trimethylsilyl-sulfid eingesetzt werden. Gegebenenfalls kann die Umsetzung auch unter sauren Bedingungen durchgeführt werden, indem als Sulfidierungsreagenzien Thioacetamid oder Thiobenzamid verwendet werden.

Die Verbindungen der allgemeinen Formel III werden hergestellt, indem man beispielsweise eine Verbindung der allgemeinen Formel IV,

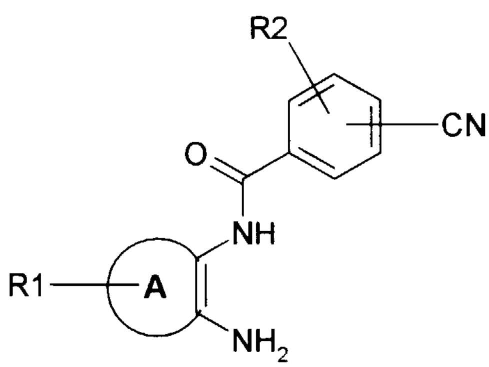

(IV)

in der $R^1$, $R^2$ und

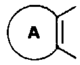

die oben angegebenen Bedeutungen besitzen, mit einer Verbindung der allgemeinen Formel V,

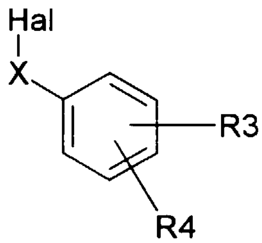

**(V)**

in der $R^3$, $R^4$ und X die oben angegebenen Bedeutungen besitzen und Hal ein Halogenatom bedeutet, in einem inerten Lösungsmittel wie z. B. Pyridin, N,N-Dimethylformamid, N-Methylpyrrolidon, Dioxan, Tetrahydrofuran, Dichlormethan oder Toluol bei Temperaturen zwischen 0°C und Siedepunkt des Lösungsmittels, vorzugsweise bei 0 bis 30°C gegebenenfalls in Gegenwart einer Base wie beispielsweise Natriumcarbonat, Kaliumcarbonat, 1,5-Diazabicyclo[5.4.0]undec-5-en, Triethylamin, N-Methylmorpholin oder Ethyldiisopropylamin oder auch in Gegenwart eines Katalysators wie z. B. 4-(Dimethylamino)-pyridin umsetzt.

Verbindungen der allgemeinen Formel III können auch hergestellt werden, indem man beispielsweise eine Verbindung der allgemeinen Formel IV, in der $R^1$, $R^2$ und

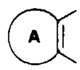

die oben angegebenen Bedeutungen besitzen, mit einer Verbindung der allgemeinen Formel VI,

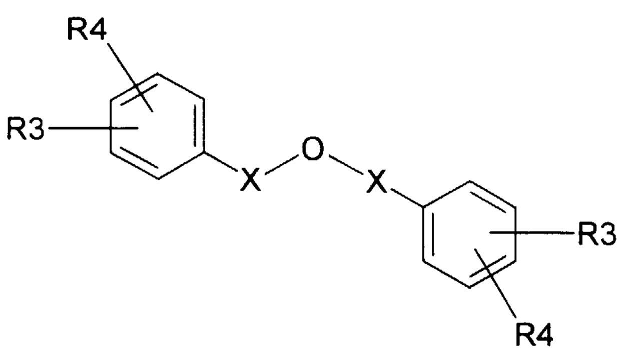

**(VI)**

in der $R^3$ und $R^4$ die oben angegebenen Bedeutungen besitzen und X eine Carbonylgruppe oder eine $SO_2$-Gruppe bedeutet, in einem inerten Lösungsmittel wie z. B. Pyridin, N,N-Dimethylformamid, N-Methylpyrrolidon, Dioxan, Tetrahydrofuran, Dichlormethan oder Toluol bei Temperaturen zwischen 0°C und Siedepunkt des Lösungsmittels, vorzugsweise bei 0 bis 30°C gegebenenfalls in Gegenwart einer Base wie beispielsweise Natriumcarbonat, Kaliumcarbonat, 1,5-Diazabicyclo[5.4.0]undec-5-en, Triethylamin, N-Methylmorpholin oder Ethyldiisopropylamin oder auch in Gegenwart eines Katalysators wie z. B. 4-(Dimethylamino)-pyridin umsetzt.

Gewisse Verbindungen der allgemeinen Formel III, in der $R^1$, $R^2$, $R^3$ und

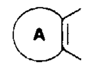

die oben angegebenen Bedeutungen besitzen, $R^4$ eine Carboxylgruppe bedeutet und X eine Carbonylgruppe bedeutet können auch hergestellt werden, indem man beispielsweise eine Verbindung der allgemeinen Formel IV, in der $R^1$, $R^2$ und

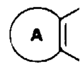

die oben angegebenen Bedeutungen besitzen, mit einer Verbindung der allgemeinen Formel VII,

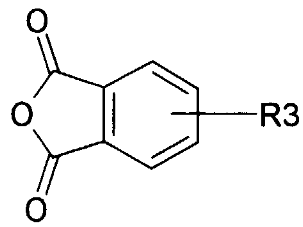

**(VII)**

in der $R^3$ die oben angegebene Bedeutungen besitzt, in einem inerten Lösungsmittel wie z. B. Pyridin, N,N-Dimethylformamid, N-Methylpyrrolidon, Dioxan, Tetrahydrofuran, Dichlormethan oder Toluol bei Temperaturen zwischen 0°C und Siedepunkt des Lösungsmittels, vorzugsweise bei 0 bis 30°C gegebenenfalls in Gegenwart einer Base wie beispielsweise Natriumcarbonat, Kaliumcarbonat, 1,5-Diazabicyclo[5.4.0]undec-5-en, Triethylamin, N-Methylmorpholin oder Ethyldiisopropylamin oder auch in Gegenwart eines Katalysators wie z. B. 4-(Dimethylamino)-pyridin zur Reaktion bringt.

Gewisse Verbindungen der allgemeinen Formel III können auch aus anderen Verbindungen der allgemeinen Formel III hergestellt werden.

Dies betrifft Verbindungen der allgemeinen Formel III, in der $R^1$, $R^2$, $R^3$, $R^4$, X und

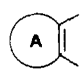

die oben angegebenen Bedeutungen besitzen, und einer oder mehrere der Reste $R^1$, $R^2$, $R^3$, $R^4$ eine Methoxygruppe bedeutet oder beinhaltet. Durch Behandlung mit gängigen, die Methylgruppe abspaltenden Reagenzien (siehe z. B.: T. W. Green, P. G. M. Wuts, "Protective Groups in Organic Synthesis", 2nd ed., John Wiley and Sons Inc. 1991) wie z. B. Trimethylsilyliodid oder Bortribromid in einem inerten Lösungsmittel wie Dichlormethan, Dichlorethan, Chloroform, Tetrachlorkohlenstoff, Tetrahydrofuran, Dioxan, Aceton, Acetonitril bei Temperaturen zwischen 0°C und dem Siedepunkt des Lösungsmittels, vorzugsweise zwischen 0°C und 60°C, können diese Verbindungen in die entsprechenden Verbindungen der allgemeinen Formel III mit freier Hydroxygruppe umgewandelt werden.

Dies betrifft auch Verbindungen der allgemeinen Formel III, in der $R^1$, $R^2$, $R^3$, $R^4$, X und

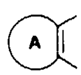

die oben angegebenen Bedeutungen besitzen, und einer oder mehrere der Reste $R^1$, $R^2$, $R^3$, $R^4$ eine Ethoxy- oder Methoxycarbonylgruppe bedeutet oder beinhaltet. Durch saure oder alkalische Hydrolyse in einem inerten Lösungsmittel wie Tetrahydrofuran, Dioxan, Aceton, Ethanol, Methanol oder Wasser bei Temperaturen zwischen 0°C und dem Siedepunkt des Lösungsmittels, vorzugsweise zwischen Raumtemperatur und 60°C, können diese Verbindungen in die entsprechenden Verbindungen der allgemeinen Formel III mit freier Carboxylgruppe umgewandelt werden.

Dies betrifft auch Verbindungen der allgemeinen Formel III, in der $R^1$, $R^2$, $R^3$, $R^4$, X und

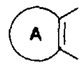

die oben angegebenen Bedeutungen besitzen, und einer oder mehrere der Reste $R^1$, $R^2$, $R^3$, $R^4$ eine Benzyloxygruppe bedeutet. Durch katalytische Hydrierung in inerten Lösungsmitteln wie z. B. Methanol, Ethanol, Tetrahydrofuran oder Dioxan in Gegenwart eines Katalysators, vorzugsweise Palladium auf Kohle, wird die Benzylgruppe dabei durch ein Wasserstoffatom ersetzt (siehe z. B.: T. W. Green, P. G. M. Wuts, "Protective Groups in Organic Synthesis", 2nd ed., John Wiley and Sons Inc. 1991). Die Entfernung der Benzylgruppe gelingt auch durch Umsetzung mit einer starken Säure wie Trifluoressigsäure in Gegenwart von Mesitylen, Anisol oder Thioanisol bei Temperaturen zwischen 0 und 50°C, vorzugsweise bei Raumtemperatur, oder durch Behandlung mit Lewissäuren wie Bortrifluorid-Etherat in einem inerten Lösungsmittel wie Toluol, Acetonitril, Diethylether oder Tetrahydrofuran bei Temperaturen zwischen 0°C und dem Siedepunkt des Lösungsmittels, vorzugsweise zwischen Raumtemperatur und dem Siedepunkt des Lösungsmittels.

Dies betrifft auch Verbindungen der allgemeinen Formel III, in der $R^1$, $R^2$, $R^3$, $R^4$, X und

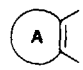

die oben angegebenen Bedeutungen besitzen, und einer oder mehrere der Reste $R^1$, $R^2$, $R^3$, $R^4$ eine Allyloxygruppe bedeutet. Durch übergangsmetallkatalysierte Spaltung, beispielsweise in Gegenwart eines Rhodiumkatalysators wie Tristriphenylphosphin-rhodiumchlorid oder eines Palladiumkatalysators wie Tetrakistriphenylphosphin-palladium in einem inerten Lösungsmittel wie Tetrahydrofuran oder Dioxan, gegebenenfalls in Anwesenheit eines Nucleophils wie z. B. Malonsäurediethylester, Tributylzinnhydrid, 5,5-Dimethyl-cyclohexan-1,3-dion oder Piperidin bei Temperaturen zwischen 0°C und 50°C, vorzugsweise bei Raumtemperatur, wird die Allylgruppe dabei durch ein Wasserstoffatom ersetzt (siehe z. B.: T. W. Green, P. G. M. Wuts, "Protective Groups in Organic Synthesis", 2nd ed., John Wiley and Sons Inc. 1991).

Verbindungen der allgemeinen Formel V, VI und VII, in der $R^3$, $R^4$, X und Hal die oben angegebenen Bedeutungen besitzen, sind entweder käuflich oder literaturbekannt oder können nach literaturbekannten Verfahren hergestellt werden.

Die Verbindungen der allgemeinen Formel IV werden hergestellt, indem mar beispielsweise eine Verbindung der allgemeinen Formel VIII,

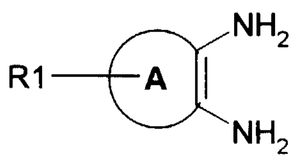

**(VIII)**

in der $R^1$ und

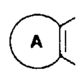

die oben angegebenen Bedeutungen besitzen, mit einer Verbindung der allgemeinen Formel IX,

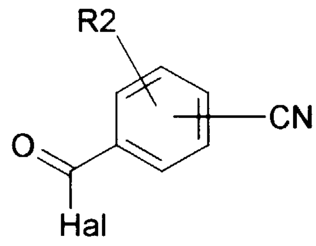

(**IX**)

in der $R^2$ die oben angegebene Bedeutung besitzt und Hal ein Halogenatom bedeutet, in einem inerten Lösungsmittel wie z. B. Pyridin, N,N-Dimethylformamid, N-Methylpyrrolidon, Dioxan, Tetrahydrofuran, Dichlormethan oder Toluol bei Temperaturen zwischen 0°C und Siedepunkt des Lösungsmittels, vorzugsweise bei 0 bis 30°C gegebenenfalls in Gegenwart einer Base wie beispielsweise Natriumcarbonat, Kaliumcarbonat, 1,5-Diazabicyclo[5.4.0]undec-5-en, Triethylamin, N-Methylmorpholin oder Ethyldiisopropylamin oder auch in Gegenwart eines Katalysators wie z. B. 4-(Dimethylamino)-pyridin umsetzt.

Verbindungen der allgemeinen Formel VIII und IX sind entweder käuflich oder literaturbekannt oder können nach literaturbekannten Verfahren hergestellt werden.

Verbindungen der allgemeinen Formel IV können auch hergestellt werden, indem man beispielsweise eine Verbindung der allgemeinen Formel X,

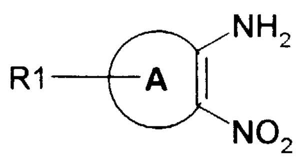

(**X**)

in der $R^1$ und

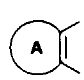

die oben angegebenen Bedeutungen besitzen, mit einer Verbindung der allgemeinen Formel IX,

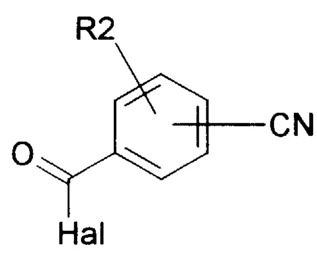

(**IX**)

in der $R^2$ die oben angegebene Bedeutung besitzt und Hal ein Halogenatom bedeutet, in einem inerten Lösungsmittel wie z. B. Pyridin, N,N-Dimethylformamid, N-Methylpyrrolidon, Dioxan, Tetrahydrofuran, Dichlormethan oder Toluol bei Temperaturen zwischen 0°C und Siedepunkt des Lösungsmittels, vorzugsweise bei 0 bis 30°C gegebenenfalls in Gegenwart einer Base wie beispielsweise Natriumcarbonat, Kaliumcarbonat, 1,5-Diazabicyclo[5.4.0]undec-5-en, Triethylamin, N-Methylmorpholin oder Ethyldiisopropylamin oder auch in Gegenwart eines Katalysators wie z. B. 4-(Dimethylamino)-pyridin umsetzt und nachfolgend die Nitrogruppe mit Reduktionsmitteln wie Zinn(II)chlorid, Titan(III)chlorid oder unedlen Metallen wie Zink, Eisen oder Nickel in Gegenwart von Säure in einem inerten Lösungsmittel wie Wasser, Methanol, Ethanol, Tetrahydrofuran, Dioxan bei Temperaturen zwischen 0°C und Siedepunkt des Lösungsmittels reduziert oder die Nitrogruppe katalytisch reduziert.

Verbindungen der allgemeinen Formel X sind entweder käuflich oder literaturbekannt oder können nach literaturbekannten Verfahren hergestellt werden.

Gewisse Verbindungen der allgemeinen Formel I lassen sich nachträglich in andere Verbindungen der allgemeinen Formel I umwandeln.

Dies betrifft Verbindungen der allgemeinen Formel I, in der $R^1$, $R^2$, $R^3$, $R^4$, X und

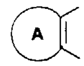

die oben angegebenen Bedeutungen besitzen, und einer oder mehrere der Reste $R^1$, $R^2$, $R^3$, $R^4$ eine Methoxygruppe bedeutet oder beinhaltet. Durch Behandlung mit gängigen, die Methylgruppe abspaltenden Reagenzien (siehe z. B.: T. W. Green, P. G. M. Wuts, "Protective Groups in Organic Synthesis", 2nd ed., John Wiley and Sons Inc. 1991) wie z. B. Trimethylsilyliodid oder Bortribromid in einem inerten Lösungsmittel wie Dichlormethan, Dichlorethan, Chloroform, Tetrachlorkohlenstoff, Tetrahydrofuran, Dioxan, Aceton, Acetonitril bei Temperaturen zwischen 0°C und dem Siedepunkt des Lösungsmittels, vorzugsweise zwischen 0°C und 60°C, können diese Verbindungen in die entsprechenden Verbindungen der allgemeinen Formel I mit freier Hydroxygruppe umgewandelt werden.

Dies betrifft auch Verbindungen der allgemeinen Formel I, in der $R^1$, $R^2$, $R^3$, $R^4$, X und

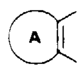

die oben angegebenen Bedeutungen besitzen, und einer oder mehrere der Reste $R^1$, $R^2$, $R^3$, $R^4$ eine Ethoxy- oder Methoxycarbonylgruppe bedeutet oder beinhaltet. Durch saure oder alkalische Hydrolyse in einem inerten Lösungsmittel wie Tetrahydrofuran, Dioxan, Aceton, Ethanol, Methanol oder Wasser bei Temperaturen zwischen 0°C und dem Siedepunkt des Lösungsmittels, vorzugsweise zwischen Raumtemperatur und 60°C, können diese Verbindungen in die entsprechenden Verbindungen der allgemeinen Formel I mit freier Carboxylgruppe umgewandelt werden.

Dies betrifft auch Verbindungen der allgemeinen Formel I, in der $R^1$, $R^2$, $R^3$, $R^4$, X und

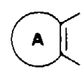

die oben angegebenen Bedeutungen besitzen, und einer oder mehrere der Reste $R^1$, $R^2$, $R^3$, $R^4$ eine Benzyloxygruppe bedeutet. Durch katalytische Hydrierung in inerten Lösungsmitteln wie z. B. Methanol, Ethanol, Tetrahydrofuran oder Dioxan in Gegenwart eines Katalysators, vorzugsweise Palladium auf Kohle, wird die Benzylgruppe dabei durch ein Wasserstoffatom ersetzt (siehe z. B.: T. W. Green, P. G. M. Wuts, "Protective Groups in Organic Synthesis", 2nd ed., John Wiley and Sons Inc. 1991). Die Entfernung der Benzylgruppe gelingt auch durch Umsetzung mit einer starken Säure wie Trifluoressigsäure in Gegenwart von Mesitylen, Anisol oder Thioanisol bei Temperaturen zwischen 0 und 50°C, vorzugsweise bei Raumtemperatur, oder durch Behandlung mit Lewissäuren wie Bortrifluorid-Etherat in einem inerten Lösungsmittel wie Toluol, Acetonitril, Diethylether oder Tetrahydrofuran bei Temperaturen zwischen 0°C und dem Siedepunkt des Lösungsmittels, vorzugsweise zwischen Raumtemperatur und dem Siedepunkt des Lösungsmittels.

Dies betrifft auch Verbindungen der allgemeinen Formel I, in der $R^1$, $R^2$, $R^3$, $R^4$, X und

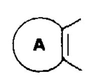

die oben angegebenen Bedeutungen besitzen, und einer oder mehrere der Reste $R^1$, $R^2$, $R^3$, $R^4$ eine Allyloxygruppe bedeutet. Durch übergangsmetallkatalysierte Spaltung, beispielsweise in Gegenwart eines Rhodiumkatalysators wie Tristriphenylphosphin-rhodiumchlorid oder eines Palladiumkatalysators wie Tetrakistriphenylphosphin-palladium in einem inerten Lösungsmittel wie Tetrahydrofuran oder Dioxan, gegebenenfalls in Anwesenheit eines Nucleophils wie z. B. Malonsäurediethylester, Tributylzinnhydrid, 5,5-Dimethyl-cyclohexan-1,3-dion oder Piperidin bei Temperaturen zwischen 0°C und 50°C, vorzugsweise bei Raumtemperatur, wird die Allylgruppe dabei durch ein Wasserstoffatom ersetzt (siehe z. B.: T. W. Green, P. G. M. Wuts, "Protective Groups in Organic Synthesis", 2nd ed., John Wiley and Sons Inc. 1991).

Zu reinen Enantiomeren der Verbindungen der Formel I kommt man entweder durch Racematspaltung (über Salzbildung mit optisch aktiven Säuren oder Basen), oder indem man in die Synthese optisch aktive Ausgangsstoffe einsetzt oder indem man enzymatisch hydrolysiert.

Bevorzugt im Sinne der Erfindung sind außer den in den Beispielen genannten Verbindungen die folgenden:

1. 6-[(Naphthalin-2-carbonyl)-amino]-5-(4-thiocarbamoyl-benzoylamino)nicotinsäuremethylester
2. 5-[(Naphthalin-2-carbonyl)-amino]-6-(4-thiocarbamoyl-benzoylamino)picolinsäuremethylester
3. 6-[(Naphthalin-2-carbonyl)-amino]-5-(4-thiocarbamoyl-benzoylamino)nicotinsäure
4. 5-[(Naphthalin-2-carbonyl)-amino]-6-(4-thiocarbamoyl-benzoylamino)picolinsäure
5. 2-(4-Methoxy-benzoylamino)-3-(4-thiocarbamoyl-benzoylamino)-pyridin
6. 3-(4-Methoxy-benzoylamino)-2-(4-thiocarbamoyl-benzoylamino)-pyridin
7. 6-(4-Methoxy-benzoylamino)-5-(4-thiocarbamoyl-benzoylamino)nicotinsäuremethylester
8. 5-(4-Methoxy-benzoylamino)-6-(4-thiocarbamoyl-benzoylamino)picolinsäuremethylester
9. 6-(3,4-Dimethoxy-benzoylamino)-5-(4-thiocarbamoyl-benzoylamino)-nicotinsäuremethylester
10. 5-(3,4-Dimethoxy-benzoylamino)-6-(4-thiocarbamoyl-benzoylamino)-picolinsäuremethylester
11. 6-(2-Carboxy-benzoylamino)-5-(4-thiocarbamoyl-benzoylamino)-nicotinsäuremethylester
12. 5-(2-Carboxy-benzoylamino)-6-(4-thiocarbamoyl-benzoylamino)-picolinsäuremethylester
13. 6-(4-Methoxy-benzolsulfonylamino)-5-(4-thiocarbamoyl-benzoylamino)-nicotinsäuremethylester
14. 5-(4-Methoxy-benzolsulfonylamino)-6-(4-thiocarbamoyl-benzoylamino)-picolinsäuremethylester
15. 6-(Naphthalin-2-sulfonylamino)-5-(4-thiocarbamoyl-benzoylamino)-nicotinsäuremethylester
16. 5-(Naphthalin-2-sulfonylamino)-6-(4-thiocarbamoyl-benzoylamino)-picolinsäuremethylester
17. 6-(Naphthalin-2-sulfonylamino)-5-(4-thiocarbamoyl-benzoylamino)-nicotinsäure
18. 5-(Naphthalin-2-sulfonylamino)-6-(4-thiocarbamoyl-benzoylamino)-picolinsäure
19. 2,3-Bis-(4-thiocarbamoyl-benzoylamino)-pyridin
20. 3-[(Naphthalin-2-carbonyl)-amino]-4-(4-thiocarbamoyl-benzoylamino)-benzoesäuremethylester
21. 3-[(Naphthalin-2-carbonyl)-amino]-4-(4-thiocarbamoyl-benzoylamino)-benzoesäure
22. 4-(4-Methoxy-benzoylamino)-3-(4-thiocarbamoyl-benzoylamino)-pyridin
23. 3-(4-Methoxy-benzoylamino)-4-(4-thiocarbamoyl-benzoylamino)-pyridin
24. 4-(3,4-Dimethoxy-benzoylamino)-3-(4-thiocarbamoyl-benzoylamino)-pyridin
25. 3-(3,4-Dimethoxy-benzoylamino)-4-(4-thiocarbamoyl-benzoylamino)-pyridin
26. 3-(3,4-Dimethoxy-benzoylamino)-2-(4-thiocarbamoyl-benzoylamino)-pyridin
27. 2-(3,4-Dimethoxy-benzoylamino)-3-(4-thiocarbamoyl-benzoylamino)-pyridin

28. 2-(3,4-Dimethoxy-benzoylamino)-1-(4-thiocarbamoyl-benzoylamino)-benzol

29. 6-(4-Methoxy-benzoylamino)-5-(4-thiocarbamoyl-benzoylamino)-nicotinsäure

30. 5-(4-Methoxy-benzoylamino)-6-(4-thiocarbamoyl-benzoylamino)-picolinsäure

31. 3-(4-Methoxy-benzoylamino)-4-(4-thiocarbamoyl-benzoylamino)-benzoesäuremethylester

32. 3-(4-Methoxy-benzoylamino)-4-(4-thiocarbamoyl-benzoylamino)-benzoesäure

33. 4-(4-Methoxy-benzoylamino)-3-(4-thiocarbamoyl-benzoylamino)-benzoesäure

34. 3-(3,4-Dimethoxy-benzoylamino)-4-(4-thiocarbamoyl-benzoylamino)-benzoesäuremethylester

35. 3-(3,4-Dimethoxy-benzoylamino)-4-(4-thiocarbamoyl-benzoylamino)-benzoesäure

36. 4-(3,4-Dimethoxy-benzoylamino)-3-(4-thiocarbamoyl-benzoylamino)-benzoesäure

37. 6-(3,4-Dimethoxy-benzoylamino)-5-(4-thiocarbamoyl-benzoylamino)-nicotinsäure

38. 5-(3,4-Dimethoxy-benzoylamino)-6-(4-thiocarbamoyl-benzoylamino)-picolinsäure

39. 4-(2-Carboxy-benzoylamino)-3-(4-thiocarbamoyl-benzoylamino)-benzoesäure

40. 4-(2,4-Dicarboxy-benzoylamino)-3-(4-thiocarbamoyl-benzoylamino)-benzoesäure

41. 4-(2,5-Dicarboxy-benzoylamino)-3-(4-thiocarbamoyl-benzoylamino)-benzoesäure

42. 4-(2,4-Dicarboxy-benzoylamino)-3-(4-thiocarbamoyl-benzoylamino)-benzoesäuremethylester

43. 4-(2,5-Dicarboxy-benzoylamino)-3-(4-thiocarbamoyl-benzoylamino)-benzoesäuremethylester

44. 4-(4-Methoxy-benzolsulfonylamino)-3-(4-thiocarbamoyl-benzoylamino)-benzoesäure

45. 3-(4-Methoxy-benzolsulfonylamino)-4-(4-thiocarbamoyl-benzoylamino)-benzoesäure

46. 3-(4-Methoxy-benzolsulfonylamino)-4-(4-thiocarbamoyl-benzoylamino)-benzoesäuremethylester

47. 6-(4-Methoxy-benzolsulfonylamino)-5-(4-thiocarbamoyl-benzoylamino)-nicotinsäure

48. 5-(4-Methoxy-benzolsulfonylamino)-6-(4-thiocarbamoyl-benzoylamino)-picolinsäure

49. 3-(Naphthalin-2-sulfonylamino)-4-(4-thiocarbamoyl-benzoylamino)-benzoesäuremethylester

50. 3-(Naphthalin-2-sulfonylamino)-4-(4-thiocarbamoyl-benzoylamino)-benzoesäure

**Beispiel 1:**

*4-[(Naphthalin-2-carbonyl)-amino]-3-(4-thiocarbamoyl-benzoylamino)-benzoesäuremethylester*

1. 3,4-Diaminobenzoesäuremethylester

In eine Lösung von 15.2 g (0.100 mol) 3,4-Diaminobenzoesäure in 200 ml Methanol wird bei Rückflußtemperatur trockenes Chlorwasserstoffgas bis zur Sättigung eingeleitet und 4 h unter Rückfluß erhitzt. Nach Abkühlen auf Raumtemperatur wird der weiße Feststoff abgetrennt, mit Diethylether gewaschen und getrocknet. Man erhält 18.2 g (76%) der Titelverbindung als Dihydrochlorid; Schmp 225-227°C (Zers.); EI-MS: 166 ($M^+$).

2. 4-Amino-3-(4-cyano-benzoylamino)-benzoesäuremethylester

Eine Lösung von 12.6 g (0.052 mol) 3,4-Diaminobenzoesäuremethylesterdihydrochlorid und 72 ml (0.520 mol) Triethylamin in 150 ml abs. Tetrahydrofuran wird bei 5°C mit einer Lösung von 8.7 g (0.052 mol) 4-Cyanobenzoylchlorid in 50 ml abs. Tetrahydrofuran versetzt und 16 h bei Raumtemperatur gerührt. Man versetzt mit Wasser, filtriert, wäscht den Rückstand nacheinander mit Wasser und Diethylether und erhält nach Trocknen 13.7 g (88%) der Titelverbindung als weißen, kristallinen Feststoff mit Schmp 202-204°C. EI-MS: 295 ($M^+$).

3. 4-[(Naphthalin-2-carbonyl)-amino]-3-(4-cyano-benzoylamino)-benzoesäuremethylester

Eine Lösung von 1.50 g (0.005 mol) 4-Amino-3-(4-cyano-benzoylamino)-benzoesäuremethylester und 10 mg (cat.) 4-Dimethylaminopyridin in 30 ml abs. Pyridin wird bei 5°C mit einer Lösung von 1.10 g (0.006 mol) 2-Naphthoylchlorid in 10 ml abs. Pyridin versetzt und 72 h bei Raumtemperatur gerührt. Man engt ein, versetzt mit Wasser und Essigsäureethylester, filtriert, wäscht den Rückstand nacheinander mit Wasser und Diethylether und erhält nach Trocknen 1.65 g (75%) der Titelverbindung als weißen, kristallinen Feststoff mit Schmp 142°C. EI-MS: 449 ($M^+$).

4. 4-[(Naphthalin-2-carbonyl)-amino]-3-(4-thiocarbamoyl-benzoylamino)-benzoesäuremethylester

In eine Lösung von 1.50 g (0.0033 mol) 4-[(Naphthalin-2-carbonyl)-amino]-3-(4-cyano-benzoylamino)-benzoesäuremethylester und 2.5 ml Triethylamin in 25 ml abs. Pyridin wird bei Raumtemperatur bis zur Sättigung Schwefelwasserstoff eingeleitet. Man rührt 6 h bei Raumtemperatur und läßt über Nacht stehen. Der ausgefallene gelbe Feststoff wird abgetrennt, mit Wasser und Diethylether gewaschen und getrocknet. Ausbeute: 1.40 g (88%), Schmp 224°C (Zers.); EI-MS: 295 ($M^+$).

**Beispiel 2:**

*4-[(Naphthalin-2-carbonyl)-amino]-3-(4-thiocarbamoyl-benzoylamino)-benzoesäure*

Eine Lösung von 0.36 ml Bortribromid (3.75 mmol) in 10 ml Methylenchlorid wird bei 5°C zu einer Lösung von 242 mg (0.50 mmol) 4-[(Naphthalin-2-carbonyl)-amino]-3-(4-thiocarbamoyl-benzoylamino)-benzoesäuremethylester in 10 ml Methylenchlorid getropft. Nach 16-stdg. Rühren bei Raumtemperatur wird die Reaktionslösung mit Eiswasser versetzt. Der ausgefallene orangefarbene Feststoff wird abgetrennt, mit Wasser und Diethylether gewaschen und getrocknet. Ausbeute: 130 mg (55%); Schmp 205°C (Zers.); (+)-LSI-MS: 470 ($MH^+$).

**Beispiel 3:**

*2-(4-Methoxy-benzoylamino)-1-(4-thiocarbamoyl-benzoylamino)-benzol*

1. 2-(4-Cyano-benzoylamino)-anilin

Eine Lösung von 2.81 g (0.026 mol) o-Phenylendiamin und 18 ml (0.130 mol) Triethylamin in 75 ml abs. Tetrahydrofuran wird bei 5°C mit einer Lösung von 4.3 g (0.026 mol) 4-Cyanobenzoylchlorid in 25 ml abs. Tetrahydrofuran versetzt und 16 h bei Raumtemperatur gerührt. Man versetzt mit Wasser, filtriert, extrahiert mit Essigester, engt ein, wäscht den Rückstand mit wenig Diethylether und erhält nach Trocknen 5.30 g (86%) der Titelverbindung als weißen, kristallinen Feststoff. EI-MS: 237 ($M^+$).

2. 2-(4-Methoxy-benzoylamino)-1-(4-thiocarbamoyl-benzoylamino)-benzol

In eine Lösung von 2.37 g (0.010 mol) 2-(4-Cyano-benzoylamino)-anilin und 10 mg (cat.) 4-Dimethylaminopyridin in 50 ml abs. Pyridin wird bei 5°C mit einer Lösung von 2.05 g (0.012 mol) 4-Methoxybenzoylchlorid in 10 ml abs. Pyridin versetzt und 16 h bei Raumtemperatur gerührt. Nach Zugabe von 10 ml Triethylamin wird bei Raumtemperatur bis zur Sättigung Schwefelwasserstoff eingeleitet. Man rührt 6 h bei Raumtemperatur, läßt über Nacht stehen, engt ein, versetzt mit 50 ml Wasser und extrahiert zweimal mit je 50 ml Essigsäureethylester. Die organische Phase wird mit 50 ml gesättigter Kochsalzlösung gewaschen, getrocknet und eingedampft. Der resultierende gelbe Feststoff wird mit wenig Diethylether gewaschen und getrocknet. Ausbeute: 3.85 g (95%); Schmp 247°C; (+)-LSI-MS: 406 ($MH^+$).

**Beispiel 4:**

*4-(4-Methoxy-benzoylamino)-3-(4-thiocarbamoyl-benzoylamino)-benzoesäuremethylester*

Eine Lösung von 0.74 g (0.0025 mol) 4-Amino-3-(4-cyano-benzoylamino)-benzoesäuremethylester und 10 mg (cat.) 4-Dimethylaminopyridin in 20 ml abs. Pyridin wird bei 5°C mit einer Lösung von 0.51 g (00030 mol) 4-Methoxybenzoylchlorid in 5 ml abs Pyridin versetzt und 16 h bei Raumtemperatur gerührt. Nach Zugabe von 2.5 ml Triethylamin wird bei Raumtemperatur bis zur Sättigung Schwefelwasserstoff eingeleitet. Man rührt 6 h bei Raumtemperatur, läßt über Nacht stehen und engt ein. Der ausgefallene gelbe Feststoff wird abgetrennt, mit Wasser und Diethylether gewaschen und getrocknet. Ausbeute: 1.00 g (91%); Schmp 208-211°C; (-)-ESI-MS: 462 ($M-H^-$).

**Beispiel 5:**

*4-(3,4-Dimethoxy-benzoylamino)-3-(4-thiocarbamoyl-benzoylamino)-benzoesäuremethylester*

Eine Lösung von 1.50 g (0.0050 mol) 4-Amino-3-(4-cyano-benzoylamino)-benzoesäuremethylester und 10 mg (cat.) 4-Dimethylaminopyridin in 30 ml abs. Pyridin wird bei 5°C mit einer Lösung von 1.50 g (0.0075 mol) 3,4-Dimethoxybenzoylchlorid in 10 ml abs. Pyridin versetzt und 16 h bei Raumtemperatur gerührt. Nach Zugabe von 5 ml Triethylamin wird bei Raumtemperatur bis zur Sättigung Schwefelwasserstoff eingeleitet. Man rührt 6 h bei Raumtemperatur, läßt über Nacht stehen und engt ein. Der ausgefallene gelbe Feststoff wird abgetrennt, mit Wasser und Diethylether gewaschen und getrocknet. Ausbeute: 2.30 g (92%); Schmp 230°C (Zers.); (+)-LSI-MS: 494 ($MH^+$).

**Beispiel 6:**

*4-(2-Carboxy-benzoylamino)-3-(4-thiocarbamoyl-benzoylamino)-benzoesäuremethylester*

Eine Lösung von 0.74 g (0.0025 mol) 4-Amino-3-(4-cyano-benzoylamino)-benzoesäuremethylester und 10 mg (cat.) 4-Dimethylaminopyridin in 25 ml abs. Pyridin wird bei 5°C mit 0.45 g (0.0030 mol) Phthalsäureanhydrid versetzt und 16 h bei Raumtemperatur gerührt. Nach Zugabe von 2.5 ml Triethylamin wird bei Raumtemperatur bis zur Sättigung Schwefelwasserstoff eingeleitet. Man rührt 6 h bei Raumtemperatur, läßt über Nacht stehen und engt ein. Der ausgefallene gelbe Feststoff wird abgetrennt, mit Wasser und Diethylether gewaschen und getrocknet. Ausbeute: 1.00 g (83%); Schmp 288-290°C; (-)-ESI-MS: 476 ($M-H^-$).

**Beispiel 7:**

*4-(4-Methoxy-benzolsulfonylamino)-3-(4-thiocarbamoyl-benzoylamino)-benzoesäuremethylester*

Eine Lösung von 0.74 g (0.0025 mol) 4-Amino-3-(4-cyano-benzoylamino)-benzoesäuremethylester und 10 mg (cat.) 4-Dimethylaminopyridin in 25 ml abs. Pyridin wird bei 5°C mit 1.28 g (0.0063 mol) 4-Methoxy-benzolsulfonylchlorid versetzt und 72 h bei Raumtemperatur gerührt. Nach Zugabe von 2.5 ml Triethylamin wird bei Raumtemperatur bis zur Sättigung Schwefelwasserstoff eingeleitet. Man rührt 6 h bei Raumtemperatur läßt über Nacht stehen, engt ein, versetzt mit 50 ml Wasser und extrahiert zweimal mit je 50 ml Essigsäureethylester. Die organische Phase wird mit 50 ml gesättigter Kochsalzlösung gewaschen, getrocknet und eingedampft. Der resultierende gelbe Feststoff wird mit wenig Diethylether gewaschen und getrocknet. Ausbeute: 0.80 g (67%); Schmp 181°C (Zers.); (+)-ESI-MS: 500 ($MH^+$).

**Beispiel 8:**

*4-(Naphthalin-2-sulfonylamino)-3-(4-thiocarbamoyl-benzoylamino)-benzoesäuremethylester*

Eine Lösung von 0.74 g (0.0025 mol) 4-Amino-3-(4-cyano-benzoylamino)-benzoesäuremethylester und 10 mg (cat.) 4-Dimethylaminopyridin in 25 ml abs. Pyridin wird bei 5°C mit 1.10 g (0.0050 mol) Naphthalin-2-sulfonylchlorid versetzt und 16 h bei Raumtemperatur gerührt. Nach Zugabe von 2.5 ml Triethylamin wird bei Raumtemperatur bis zur Sättigung Schwefelwasserstoff eingeleitet. Man rührt 6 h bei Raumtemperatur, läßt über Nacht stehen, engt ein, versetzt mit 50 ml Wasser und extrahiert zweimal mit je 50 ml Essigsäureethylester. Die organische Phase wird mit 50 ml gesättigter Kochsalzlösung gewaschen, getrocknet und eingedampft. Der resultierende gelbe Feststoff wird mit wenig Diethylether gewaschen und getrocknet. Ausbeute: 1.20 g (92%); Schmp 152°C (Zers.); (+)-ESI-MS: 500 ($MNa^+$).

**Beispiel 9:**

*1,2-Bis-(4-thiocarbamoyl-benzoylamino)-benzol*

1. 1,2-Bis-(4-cyano-benzoylamino)-benzol

Eine Lösung von 1.08 g (0.010 mol) o-Phenylendiamin und 14 ml (0.100 mol) Triethylamin in 100 ml abs. Tetrahydrofuran wird bei 5°C mit einer Lösung von 4.3 g (0.026 mol) 4-Cyanobenzoylchlorid in 25 ml abs. Tetrahydrofuran versetzt und 16 h bei Raumtemperatur gerührt. Man versetzt mit Wasser, filtriert, extrahiert mit Essigester, engt ein, wäscht den Rückstand mit wenig Diethylether und erhält nach Trocknen 2.75 g (75%) der Titelverbindung als weissen, kristallinen Feststoff. EI-MS: 366 ($M^+$).

2. 1,2-Bis-(4-thiocarbamoyl-benzoylamino)-benzol

In eine Lösung von 1.20 g (0.0033 mol) 1,2-Bis-(4-cyano-benzoylamino)-benzol und 2.5 ml Triethylamin in 25 ml abs. Pyridin wird bei Raumtemperatur bis zur Sättigung Schwefelwasserstoff eingeleitet. Man rührt 6 h bei Raumtemperatur und läßt über Nacht stehen und engt ein. Der ausgefallene gelbe Feststoff wird abgetrennt, mit Wasser und Diethylether gewaschen und getrocknet. Ausbeute: 0.96 g (69%); Schmp 292-294°C; (+)-FAB-MS: 435 ($MH^+$).

**Beispiel 10:**

*4-(Naphthalin-2-sulfonylamino)-3-(4-thiocarbamoyl-benzoylamino)-benzoesäure*

Eine Lösung von 0.24 ml Bortribromid (2.5 mmol) in 10 ml Methylenchlorid wird bei 5°C zu einer Lösung von 260 mg (0.5 mmol) 4-(Naphthalin-2-sulfonylamino)-3-(4-thiocarbamoyl-benzoylamino)-benzoesäuremethylester in 10 ml Methylenchlorid getropft. Nach 16-stdg. Rühren bei Raumtemperatur wird die Reaktionslösung mit Eiswasser versetzt. Der ausgefallene orangefarbene Feststoff wird abgetrennt, mit Wasser und Diethylether gewaschen und getrocknet. Ausbeute: 120 mg (48%); Schmp 215°C (Zers.); (+)-ESI-MS: 528 ($MNa^+$).

**Beispiel 11:**

Pharmakologische Versuchsbeschreibung

Plasmagewinnung

Neun Teile frisches Blut gesunder Spender werden mit einem Teil Natriumcitratlösung (0.11 Mol/l) gemischt und bei ca. 3000 U/min 10 Minuten bei Raumtemperatur zentrifugiert. Das Plasma wird abpipettiert und kann bei Raumtemperatur ca. 8 h aufbewahrt werden.

Aktivierte partielle Thromboplastinzeit (APTT)

100 µl Citratplasma und 100 µl APTT-Reagenz (Diagnostica Stago / Boehringer Mannheim GmbH; enthält Lyophilisat Cephalin mit mikrokristallinem Kieselguraktivator) werden zusammen mit 10 µl Dimethylsulfoxid (DMSO) oder 10 µl einer Lösung der Wirksubstanz in DMSO in einem Kugelkoagulometer (KC10 der Fa. Amelung) 3 Minuten bei 37°C inkubiert. Mit Zugabe von 100 µl 0.025 M Calciumchlorid-Lösung wird eine Stoppuhr gestartet und der Zeitpunkt bis zum Eintritt der Gerinnung bestimmt. Die APTT beträgt bei den Kontrollmessungen ca. 28 -35 Sekunden und wird durch Wirksubstanzen verlängert. Sofern bei den Messungen nach 5 Minuten keine Gerinnung eintrat, wurde der Versuch gestoppt (>300).

In Tabelle 1 sind die gemessenen APTT-Zeiten in Sekunden als Differenz zur Kontrolle angegeben. Die Konzentrationen der Wirksubstanzen betrugen im Endvolumen 1000 µM (APTT 1000), 100 µM (APTT 100), 10 µM (APTT 10), 1 µM (APTT 1).

Thrombinzeit

200 µl Citratplasma werden in einem Kugelkoagulometer (KC10 der Fa. Amelung) 2 Minuten bei 37°C inkubiert. Zu 190 µl vortemperiertem Thrombin-Reagenz (Boehringer Mannheim GmbH; enthält ca. 3 U/ml Pferdethrombin und 0.0125 M $Ca^{++}$ gibt man 10 µl Dimethylsulfoxid (DMSO) oder eine Lösung der Wirksubstanz in DMSO. Mit Zugabe dieser 200 µl Lösung zum Plasma wird eine Stoppuhr gestartet und der Zeitpunkt bis zum Eintritt der Gerinnung

bestimmt. Die Thrombinzeit beträgt bei den Kontrollmessungen ca. 24 Sekunden und wird durch Wirksubstanzen verlängert. Sofern bei den Messungen nach 5 Minuten keine Gerinnung eintrat, wurde der Versuch gestoppt (>300).

In Tabelle 1 sind die gemessenen Thrombinzeiten in Sekunden als Differenz zur Kontrolle angegeben. Die Konzentrationen der Wirksubstanzen betrugen im Endvolumen 500 µM (TT 500).

Inhibitionskonstanten

Die kinetischen Messungen wurden in 0.2 M Kochsalz und 0.5 % Polyethylenglycol 6000 enthaltendem 0.1 M Phosphatpuffer (Herstellung siehe unten) bei pH 7.5 und 25°C in Polystyrol-Halbmikroküvetten in einem Gesamtvolumen von 1 mi durchgeführt. Die Reaktionen wurden durch Zugabe von Enzym zu vorinkubierten Lösungen, die entweder Dimethylsulfoxid (Kontrolle) oder Lösungen der Testsubstanz in DMSO (Inhibitor-Stocklösungen: 10 mM in DMSO) enthielten, gestartet. Die Zunahme der Extinktion bei 405 nm als Folge der Freisetzung von 4-Nitroanilin aus dem Substrat wurde photometrisch über einen Zeitraum von 12 Minuten verfolgt. Im Abstand von 20 Sekunden wurden Meßwerte (Extinktion vs. Zeit) ermittelt und diese Daten per Computer gespeichert.

Zur Bestimmung der Inhibitionskonstanten $K_i$ wurde wie folgt vorgegangen: Die Geschwindigkeiten $V_0$ (Extinktionsänderung pro Sekunde; Messungen ohne Inhibitor) und $V_i$ (Extinktionsänderung pro Sekunde; Messungen mit Inhibitor) wurden durch lineare Regression bestimmt, wobei nur die Meßpunkte berücksichtigt wurden, bei der sich die Substratkonzentration um weniger als 15% verringerte. Aus einer Meßreihe (konstante Inhibitorkonzentration, variable Substratkonzentrationen) bestimmte man $K_M$ und $V_{MAX}$ durch nichtlinearen Fit auf die Gleichung

$$V = \frac{V_{max} * [S]}{[S] + K_M}$$

Aus den geseamten Meßreihen mit 16 Datensätzen (Messungen bei 4 verschiedene Substratkonzentrationen und jeweils 4 verschiedenenen Inhibitorkonzentrationen) erhält man den $K_i$-Wert durch nichtlineare Regression aus der Gleichung

$$V_i = \frac{V_{max} * [S]}{K_M * (1 + [I]/K_i) + [S]}$$

wobei $V_{Max}$ die maximale Geschwindigkeit in Abwesenheit eines Inhibitors, $K_M$ die Michaliskonstante und [S] die Substratkonzentration bedeuten.

In Tabelle 1 sind die gemessenen $K_i$-Werte in [µM] angegeben.

FXa:

Stocklösung: 990 µl Phophatpuffer-Lösung (Herstellung siehe unten) wurden mit 10 µl humanem Faktor Xa (Boehringer Mannheim GmbH; 10 U; Suspension) versetzt und auf Eis maximal 4 Stunden gelagert. Zur Messung werden 850 µl Phosphatpuffer mit 100 µl Substrat [N-Methoxycarbonyl-(D)-norleucyl-glycyl-(L)-arginin-4-nitroanilin-Acetat; Chromozym X; Boehringer Mannheim GmbH; verwendete Substratkonzentrationen 800, 600, 400 und 200 µM; $K_M$ 400 µM] und 25 µl Inhibitor-Lösung oder 25 µl DMSO (Kontrolle) im Photometer thermostatiert (25°C). Durch Zugabe von 25 µl Stocklösung wird die Reaktion gestartet.

Thrombin:

Humanes α-Thrombin (Sigma; 100 U; spezifische Aktivität: 2000 NIH-units/mg) wird in 1 ml Wassser gelöst und in Portionen zu 20 µl bei - 18°C gelagert. Stocklösung: 1480 µl Phophatpuffer-Lösung (Herstellung siehe unten) wurden mit 20 µl der wie voranstehend hergestellten α-Thrombin-Lösung versetzt und auf Eis maximal 4 Stunden gelagert. Zur Messung werden 850 µl Phosphatpuffer mit 100 µl Substrat [H-(D)-Phe-Pip-Arg-4-nitroanilin-dihydrochlorid; S-2238; Kabi; verwendete Substratkonzentrationen 100, 50, 30 und 20 µM; $K_M$ 4 µM) und 25 µl Inhibitor-Lösung oder 25 µl DMSO (Kontrolle) im Photometer thermostatiert (25°C). Durch Zugabe von 25 µl Stocklösung wird die Reaktion gestartet.

Trypsin:

10 mg bovines pankreatisches Trypsin (Sigma) wird in 100 ml 1mM Salzsäure gelöst und im Kühlschrank bei

2-8°C aufbewahrt. Stocklösung: 990 µl 1 mM Salzsäure wurden mit 10 µl der wie voranstehend hergestellten Trypsin-Lösung versetzt und auf Eis maximal 4 Stunden gelagert. Zur Messung werden 850 µl Phosphatpuffer mit 100 µl Substrat [H-(D)-Phe-Pip-Arg-4-nitroanilin-dihydrochlorid; S-2238; Kabi; verwendete Substratkonzentrationen 100, 50, 30 und 20 µM; $K_M$ 45 µM) und 25 µl Inhibitor-Lösung oder 25 µl DMSO (Kontrolle) im Photometer thermostatiert (25°C). Durch Zugabe von 25 µl Stocklösung wird die Reaktion gestartet.

Herstellung der 0.1M Phosphatpuffer-Lösung (pH 7.5, 0.2 M NaCl):

8.90 g $Na_2HPO_4 \cdot 2\ H_2O$, 5.84 g NaCl und 2.50 g Polyethylenglykol 6000 werden in 400 ml destilliertem Wasser gelöst und mit destilliertem Wasser auf ein Gesamtvolumen von 500 ml aufgefüllt (Lösung I). 1.36 g $KH_2PO_4$, 1.17 g NaCl und 0.50 g Polyethylenglykol 6000 werden in 80 ml destilliertem Wasser gelöst und mit destilliertem Wasser auf ein Gesamtvolumen von 100 ml aufgefüllt (Lösung II). Dann gibt man soviel Lösung II (ca. 85 ml) zu Lösung I bis der pH-Wert 7.5 beträgt. Die Pufferlösung wird stets frisch hergestellt (bei Lagerung im Kühlschrank bei 4°C maximal 10 Tage haltbar).

Tabelle 1

| Pharmakologische Daten der Beispielverbindungen 1-10 | | | | | | | | |
|---|---|---|---|---|---|---|---|---|
| Beispiel Nr. | Ki [µM] FXa | Ki [µM] Thrombin | Ki [µM] Trypsin | APTT 1000 [sec] | APTT 100 [sec] | APTT 10 [sec] | APTT 1 [sec] | TT 500 [sec] |
| **1** | | | | 1 | | | | |
| **2** | | | | 9.4 | | | | |
| **3** | 0.050 | >100 | >100 | 157.9 | 32.4 | 8.4 | 2.6 | 2.4 |
| **4** | | | | 65.3 | | | | |
| **5** | | | | 3.5 | | | | |
| **6** | | | | 9.0 | | | | |
| **7** | | | | 0.8 | | | | |
| **8** | | | | -0.4 | | | | |
| **9** | | | | 6.7 | | | | |
| **10** | | | | 0.7 | | | | |

## Patentansprüche

1. Verbindungen der allgemeinen Formel I

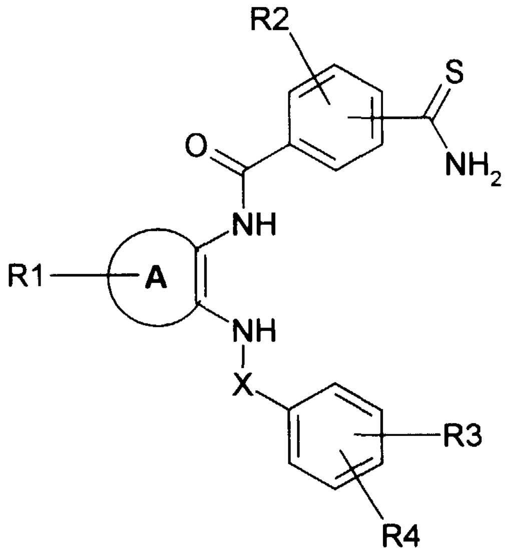

(I)

in der

R[1] ein Wasserstoffatom, ein Halogenatom, eine Hydroxygruppe, eine Aminogruppe, eine Nitrogruppe, eine Carboxylgruppe, eine Carbamoylgruppe, eine Thiocarbamoylgruppe, eine Alkylgruppe, eine Cycloalkylgruppe, eine Alkenylgruppe, eine Alkinylgruppe, eine Alkoxygruppe, eine Hydroxyalkylgruppe, eine Alkoxyalkylgruppe, eine Aralkyloxygruppe, eine Alkenyloxygruppe, eine Alkinyloxygruppe, eine Carboxyalkylgruppe, eine Alkoxycarbonylgruppe, eine Alkenyloxycarbonylgruppe, eine Alkinyloxycarbonylgruppe, eine Alkyloxycarbonylalkylgruppe, eine Alkenyloxycarbonylalkylgruppe oder eine Alkinyloxycarbonylalkylgruppe sein können;

R[2] ein Wasserstoffatom, ein Halogenatom, eine Hydroxygruppe, eine Aminogruppe, eine Nitrogruppe, eine Carboxylgruppe, eine Carbamoylgruppe, eine Alkylgruppe, eine Cycloalkylgruppe, eine Alkenylgruppe, eine Alkinylgruppe, eine Alkoxygruppe, eine Hydroxyalkylgruppe, eine Alkoxyalkylgruppe, eine Aralkyloxygruppe, eine Alkenyloxygruppe, eine Alkinyloxygruppe, eine Carboxyalkylgruppe, eine Alkoxycarbonylgruppe, eine Alkenyloxycarbonylgruppe, eine Alkinyloxycarbonylgruppe, eine Alkyloxycarbonylalkylgruppe, eine Alkenyloxycarbonylalkylgruppe oder eine Alkinyloxycarbonylalkylgruppe sein können;

$R^3$, $R^4$ gleich oder verschieden sind und ein Wasserstoffatom, ein Halogenatom, eine Hydroxygruppe, eine Aminogruppe, eine Nitrogruppe, eine Carboxylgruppe, eine Carbamoylgruppe, eine Thiocarbamoylgruppe, eine Alkylgruppe, eine Cycloalkylgruppe, eine Alkenylgruppe, eine Alkinylgruppe, eine Alkoxygruppe, eine Hydroxyalkylgruppe, eine Alkoxyalkylgruppe, eine Aralkyloxygruppe, eine Alkenyloxygruppe, eine Alkinyloxygruppe, eine Carboxyalkylgruppe, eine Alkoxycarbonylgruppe, eine Alkenyloxycarbonylgruppe, eine Alkinyloxycarbonylgruppe, eine Alkyloxycarbonylalkylgruppe, eine Alkenyloxycarbonylalkylgruppe oder eine Alkinyloxycarbonylalkylgruppe sein können oder $R^3$ und $R^4$ zusammen mit dem Arylrest, an den sie gebunden sind einen kondensierten Aromaten wie einen Naphthylrest, einen Chinolinrest oder einen Isochinolinrest bilden;

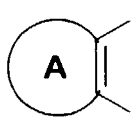

eines der aromatischen Fragmente Phenylen, Thiophen-2,3-diyl, Thiophen-3,4-diyl, Furan-2,3-diyl, Furan-3,4-diyl, Pyridin-2,3-diyl, Pyridin-3,4-diyl, Pyridin-5,6-diyl, Pyridazin-3,4-diyl oder Pyridazin-4,5-diyl bedeutet und

X eine geradkettige oder verzweigte Alkylengruppe, eine Carbonylgruppe oder eine $SO_2$-Gruppe bedeutet,

sowie deren Hydrate, Solvate und physiologisch verträgliche Salze, optisch aktive Formen, Racemate und Diastereomerengemische.

**2.** Verbindungen gemäß Anspruch 1, in denen

$R^1$ ein Wasserstoffatom, ein Chloratom, eine Hydroxygruppe, eine Carboxylgruppe, eine Methylgruppe, eine Ethylgruppe, eine *tert.*-Butylgruppe, eine Methoxygruppe, eine Ethoxygruppe, eine *tert.*-Butyloxygruppe, eine Benzyloxygruppe, eine Allyloxygruppe, eine Methoxycarbonylgruppe, eine Ethoxycarbonylgruppe oder eine Allyloxycarbonylgruppe bedeuten;

$R^2$ ein Wasserstoffatom ein Chloratom, eine Hydroxygruppe, eine Carboxylgruppe, eine Thiocarbamoylgruppe, eine Methylgruppe, eine Ethylgruppe, eine *tert.*-Butylgruppe, eine Methoxygruppe, eine Ethoxygruppe, eine *tert.*-Butyloxygruppe, eine Benzyloxygruppe, eine Allyloxygruppe, eine Methoxycarbonylgruppe, eine Ethoxycarbonylgruppe oder eine Allyloxycarbonylgruppe bedeuten;

$R^3$, $R^4$ gleich oder verschieden sind und ein Wasserstoffatom, ein Chloratom, eine Hydroxygruppe, eine Carboxylgruppe, eine Thiocarbamoylgruppe, eine Methylgruppe, eine Ethylgruppe, eine *tert.*-Butylgruppe, eine Methoxygruppe, eine Ethoxygruppe, eine *tert.*-Butyloxygruppe, eine Benzyloxygruppe, eine Allyloxygruppe, eine Methoxycarbonylgruppe, eine Ethoxycarbonylgruppe oder eine Allyloxycarbonylgruppe bedeuten oder $R^3$ und $R^4$ zusammen mit dem Arylrest an den sie gebunden sind einen Naphthylrest bilden;

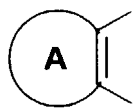

eines der aromatischen Fragmente Phenylen, Pyridin-2,3-diyl, Pyridin-3,4-diyl, Pyridin-5,6-diyl bedeutet und

X eine Carbonylgruppe oder eine $SO_2$-Gruppe bedeutet, sowie deren Hydrate, Solvate und physiologisch verträgliche Salze, optisch aktive Formen, Racemate und Diastereomerengemische.

**3.** Pharmazeutische Zubereitungen, enthaltend mindestens eine Verbindung der Formel I gemäß Anspruch 1 oder 2 neben üblichen Träger- und Hilfsstoffen.

**4.** Verwendung von Verbindungen der Formel I gemäß Anspruch 1 oder 2 zur Herstellung von Arzneimitteln mit antithromboembolischer Wirkung.

Europäisches Patentamt

# EUROPÄISCHER RECHERCHENBERICHT

Nummer der Anmeldung
EP 98 10 2751

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,A | T. NAGAHARA, ET AL.: "Dibasic (amidinoaryl)propanoic acid derivatives as novel blood coagulation factor Xa inhibitors" JOURNAL OF MEDICINAL CHEMISTRY, Bd. 37, Nr. 8, 15.April 1994, WASHINGTON, DC, US, Seiten 1200-1207, XP000608128 * das ganze Dokument * | 1-4 | C07C327/48<br>A61K31/165<br>A61K31/18 |
| D,A | R.R. TIDWELL, ET AL.: "Strategies for anticoagulation with synthetic protease inhibitors. Xa inhibitors versus thrombin inhibitors" THROMBOSIS RESEARCH, Bd. 19, Nr. 3, 1.August 1980, TARRYTOWN, US, Seiten 339-349, XP000574196 * das ganze Dokument * | 1-4 | |
| A | US 5 612 363 A (R. MOHAN, ET AL.) 18.März 1997 * das ganze Dokument * | 1-4 | **RECHERCHIERTE SACHGEBIETE (Int.Cl.6)**<br>C07C<br>C07D<br>A61K |

Der vorliegende Recherchenbericht wurde für alle Patentansprüche erstellt

| Recherchenort | Abschlußdatum der Recherche | Prüfer |
|---|---|---|
| DEN HAAG | 1.Juli 1998 | English, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 98 10 2751

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-07-1998

| Im Recherchenbericht angeführtes Patentdokument | Datum der Veröffentlichung | Mitglied(er) der Patentfamilie | Datum der Veröffentlichung |
|---|---|---|---|
| US 5612363 A | 18-03-1997 | AU 5974696 A | 18-12-1996 |
| | | EP 0848708 A | 24-06-1998 |
| | | WO 9638421 A | 05-12-1996 |
| | | US 5726173 A | 10-03-1998 |
| | | US 5731308 A | 24-03-1998 |
| | | US 5731311 A | 24-03-1998 |
| | | US 5726198 A | 10-03-1998 |
| | | US 5728697 A | 17-03-1998 |

EPO FORM P0461

Für nähere Einzelheiten zu diesem Anhang : siehe Amtsblatt des Europäischen Patentamts, Nr.12/82